# EUROPEAN PATENT APPLICATION

(11) **EP 3 460 806 A1**
(43) Date of publication of application: **27.03.2019**
(21) Application number: 17193068.8
(22) Date of filing: 26.09.2017
(51) Int. Cl.: G16H 50/30

(54) **A COMPUTER-IMPLEMENTED METHOD OF PREDICTING AND ALERTING A HEALTH RISK IN AN AGGREGATED CLINICAL CAREPATH**

(71) Applicant: AGFA Healthcare, 2640 Mortsel (BE)
(72) Inventor: Sun, Hong, 2640 Mortsel (BE); Depraetere, Kristof, 2640 Mortsel (BE); Felix, Joost, 2640 Mortsel (BE); De Roo, Jos, 2640 Mortsel (BE)
(74) Representative: Verbrugghe, Anne Marie L.

(57) **Abstract**

A computer-implemented method of predicting and alerting a health risk in an aggregated clinical carepath prescribing a number of state transitions resulting from medical interventions, by simulating said state transitions in sequence and checking whether an abnormal situation requiring an alert exists. If an alert situation is detected, generating said alert; if no alert situation is detected, continuing to simulate next transitions of said aggregated carepath and again checking whether an abnormal situation occurs, repeating detection steps and transition steps until all state transitions of said aggregated carepath have been performed.

## Description

### FIELD OF THE INVENTION

The present invention relates to clinical (care)paths and more specifically relates to a method to predict risks in integrated clinical (care)paths.

### BACKGROUND OF THE INVENTION

A clinical path (carepath, clinical pathway) consists of a set of treatments and/or actions or interventions to be performed by professionals involved in medical treatment to help a patient to move from a current health state to a target health state, more specifically from a state in which the patient has a disease to a state in which that disease is cured or controlled.

The treatment plan made in the clinical path is mostly based on the current state deduced from biometric data of the patient and the disease to be tackled. In the situation like comorbidity, there exist several clinical paths to cope with different diseases.

The problem is that a planned action in one path could change the state of a patient in the future, and that such a change is not explicit to another path (which is only produced based on the current state), and in some cases, might be considered as a risk or alarm for another path.

While some of such conflicts can be detected by stating certain actions as conflicts, e.g. contraindication, others are not so obvious when there is no stated conflicts between the actions, but only the consequence of one action, under certain condition (state), causes an unacceptable state for another action.

It is an aspect of the present invention to overcome the above-described problems.

### SUMMARY OF THE INVENTION

The above-mentioned aspects are realized by a method having the specific method steps set out in claim 1.

Specific features for preferred embodiments of the invention are set out in the dependent claims.

This invention introduces a method that can predict risks in clinical pathway, not only based on the current state, but also on a state change following actions planned in the clinical carepaths, thus allowing to predict risks in the future.

Further advantages and embodiments of the present invention will become apparent from the following description and drawings.

### BRIEF DESCRIPTION OF THE DRAWINGS

Fig. 1 illustrates State transition in Linear State Transition Logic,
Fig. 2 shows clinical path paths represented with state transitions,
Fig. 3 illustrates the execution of the state transitions,
Fig. 4 - 7 illustrate the method applied to a specific example, more in particular to patient profiles that are subjected to a diabetes treatment,
Fig. 8 is a Turtle representation of patient 1,
Fig. 9 schematically represents the target to be reached,
Fig. 10 is the N3 representation of this target,
Fig. 11 illustrates the state transition as a consequence of administering Metformin,
Fig. 12 is a table representing the state transition description,
Fig. 13 shows different alarms: overweight, underweight and Metformin Disease Interaction,
Fig. 14 is an N3 representation of the underweight alarm,
Fig. 15 are SNOWMED hierarchical rules,
Fig. 16 shows the BMI calculation rule,
Fig. 17 shows a detected overweight alert,

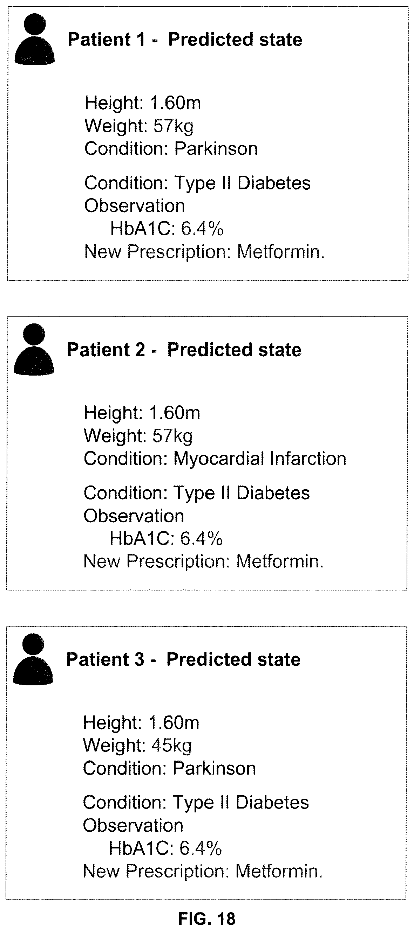

Fig. 18 shows predicted states for each of patients 1 to 3,
Fig. 19 illustrates a sample warning for patient 2,
Fig. 20 shows a sample warning for patient 3.

### DETAILED DESCRIPTION OF THE INVENTION

A clinical care path comprises a set of treatments and /or actions that help a patient to move from a current state in which the patient has a disease to a target state in which the disease is cured or controlled.

The sequence to execute the actions is explicit, however the consequence of taking the actions are often implicit. Explicitly state the consequence of an action would allow us to formally express the expected state change introduced by taking an action.

Once all the actions contained in a clinical care path have corresponding descriptions that explicitly state their consequences, the state transition, which evolves from the current state to the target state, would also become explicit.

Classical and intuitionistic logic processes the state change as follows:

| |
|---|
| If A and A => B, then B, *but A still holds* |

This is correct in mathematics, but could be wrong in real-life, e.g. in clinical applications.

For example:

| |
|---|
| Let A be the fact that patient X has temperature 40 °C |

| |
|---|
| Let A=>B be the consequence of taking pill Paracetamol, that temperature drop from 40 °C (A) to 37 °C (B). |

Then given A and A=>B (i.e. patient X has temperature 40 °C and takes a medicament containing Paracetamol), following the classical and intuitionistic logic, the consequence would be both fact A and fact B. The patient has a temperature of both 40 °C and 37 °C, while in reality, only the latter is required for further care and the former one is not needed any more.

Linear Logic solves this problem by eliminating the previous state. In Linear Logic [Reference: Girard J Y. Linear logic: its syntax and semantics[J]. London Mathematical Society Lecture Note Series, 1995: 1-42.], the state change is expressed as below, the fact on the left side of the transition will be consumed, and does not hold any more.

| |
|---|
| If A and A 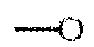 B, then B, *and A does not hold any more* |

Taking the aforementioned example:

| |
|---|
| Let A be the fact that patient X has temperature 40 °C |

| |
|---|
| Let A 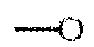 B be the consequence of taking pill Paracetamol, that temperature drop from 40 °C (A) to 37 °C (B). |

Then given A and A 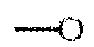 B (i.e. patient X has temperature 40 °C and takes a medicament containing Paracetamol), following Linear Logic, the consequence would be only fact B. The patient has a temperature of 37 °C, and the fact that the patient has a temperature of 40 °C will be dropped.

However, Linear Logic also has its own problem in the case below, where the text in grey is intended to state a condition that patient X should have no contraindication with Paracetamol:

| |
|---|
| Let A be the fact that patient X has temperature 40 °C, and patient X has no contraindication with Paracetamol. |

| |
|---|
| Let A 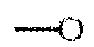 B be the consequence of taking pill Paracetamol, that temperature drop from 40 °C (A) to 37 °C (B). |

Then given A and A 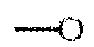 B (i.e. patient X has temperature 40 °C and takes a medicament containing Paracetamol), following linear logic, the consequence would be only fact B. The patient has a temperature of 37 °C. However the fact that the patient has no contraindication to Paracetamol will be dropped as well.

This is contradictory to the purpose that this fact should work as a condition to perform the action.

If Paracetamol is still required later, dropping the fact (patient X has no contraindication to Paracetamol) would prohibit administering Paracetamol to patient X, because one of the conditions to prescribe Paracetamol fails to be present. This is an unexpected result.

This problem is solved in the present invention by implementing a Linear State Transition Logic which explicitly separates conditions from facts.

The following example explains this:

| |
|---|
| Given Fact A, Condition C, apply Transition A 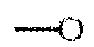 B, then B, *and A does not hold any more, and C may still hold.* |

In Linear State Transition Logic, the condition may still hold after applying the transition. The Linear State Transition Logic solves the aforementioned issue as follows:

| |
|---|
| Let A be the fact that patient X has temperature 40 °C. |

| |
|---|
| Let C be the condition that patient X has no contraindication with Paracetamol. |

| |
|---|
| Let A 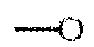 B be the consequence of taking pill Paracetamol, that temperature drop from 40 °C (A) to 37 °C (B). |

Then given A, C, and A 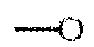 B, following state transition logic, the consequence would be only fact B. The patient is with temperature 37 °C. However, the condition that the patient has no contraindication with Paracetamol still holds.

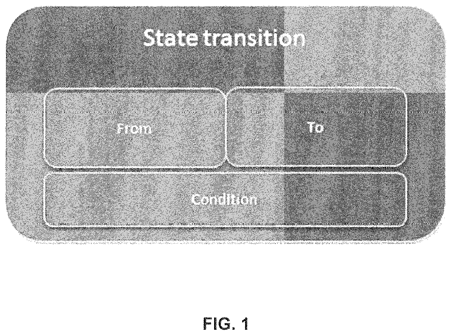

Figure 1 schematically illustrates the description of a state transition in the proposed Linear State Transition Logic. The 'From' indicates the facts to be retracted. The 'To' indicates the facts to be asserted. The 'Condition' indicates the condition to carry out the state transition that extracts the 'From' facts, and asserts the 'To' facts.

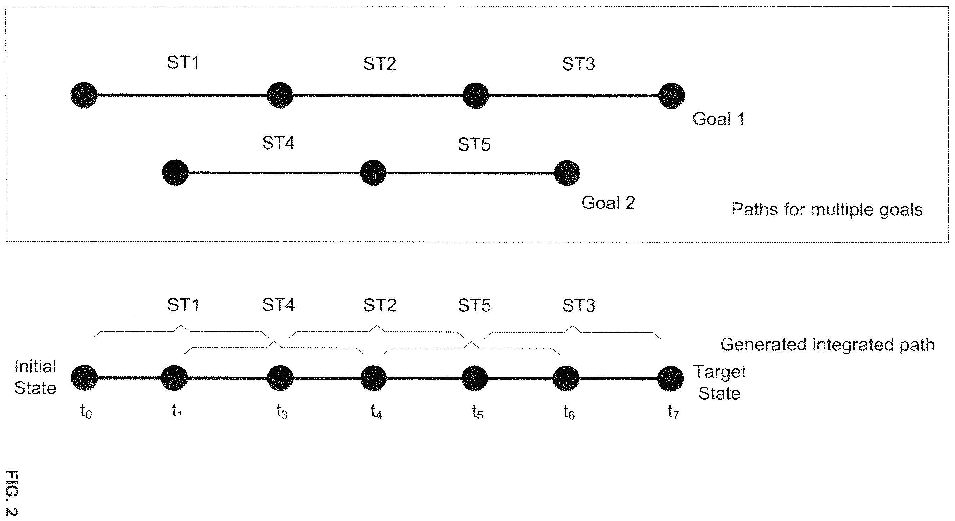

Figure 2 shows clinical path paths represented with state transitions. The upper pane shows two separated paths that reaches two separate goals. Goal 1 is expected to be reached by executing state transition ST1, ST2, and ST3 in sequence, and Goal 2 is expected to be reached by executing state transition ST4 and ST5 in sequence.

The lower pane shows the aggregated path. Based on the time point of each state transition indicated in the upper pane, these state transitions are aggregated together into one aggregated path. It is expected that by executing the state transition ST1, ST4, ST2, ST5, and ST3 in sequence, both goal 1 and goal 2 can be reached.

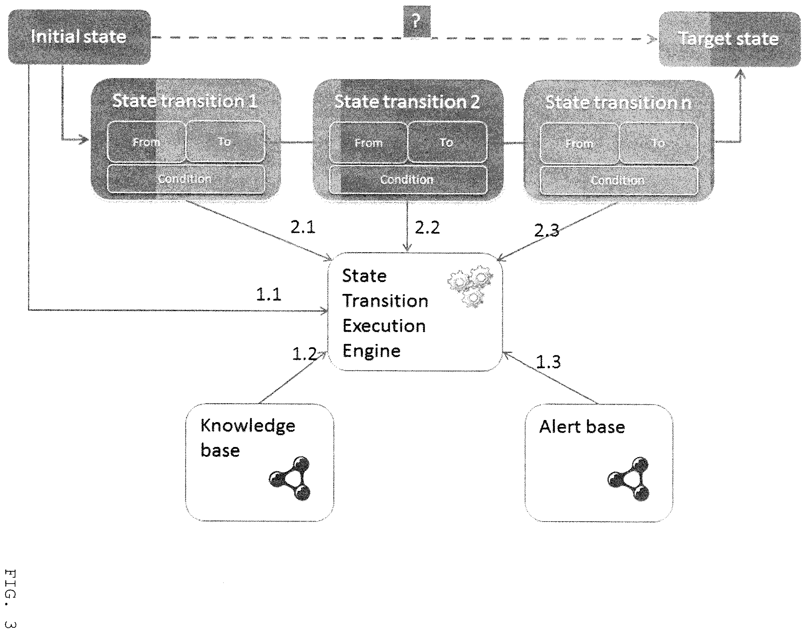

Figure 3 illustrates the execution of state transitions.

Once an aggregated path as indicated in Figure 3 is generated, a state transition execution engine would execute the state transitions in sequence to simulate the predicted state transition.

During the simulation of state change, if an alert situation is predicted, the engine will generate an alert and provide the trace that leads to the detected situation.

Figure 3 illustrates how the state transition execution engine executes state transitions and detects alert.

The state transition execution engine first imports the initial state of a patient, as well as the knowledge base and alert base.

The initial state reflects the current state of a patient without introducing any state transition.

Semantic web language is used to describe the initiate state.

The knowledge base allows to infere additional knowledge from the known state. The knowledge base comprises said the aggregated care path and a set of state transition descriptions. Semantic web language is used to describe medical knowledge as facts, as well as rules.

The alert base describes abnormal situations that are expected to be detected. Semantic web language is used to describe alerts as rules.

After the initial state, knowledge base and alert base are loaded, the state transition execution engine will execute the alert detection rules, if any of the alert rule is triggered, it indicates there is an alert of the current state of the patient.

If no alert is detected, the engine will carry the first state transition listed in the aggregated path, by extracting the stated 'From' pattern and asserting the stated 'To' pattern.

After finishing a state transition, the state transition execution engine will re-run the alert detection rules.

If any of the alert rule is triggered, it indicates that according to the state transition description, it is predicted that an abnormal situation would happen in future.

If none of the alert rules is triggered, the state transition execution engine will execute the next state transition and reexecute the alert detection rules. This is repeated until the state transition execution engine reaches the end of the aggregated path.

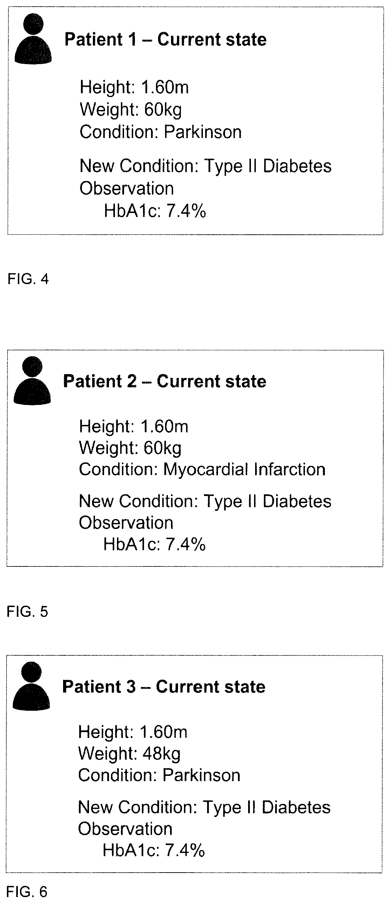

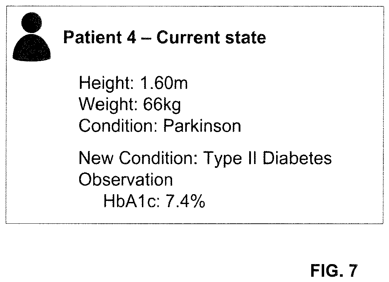

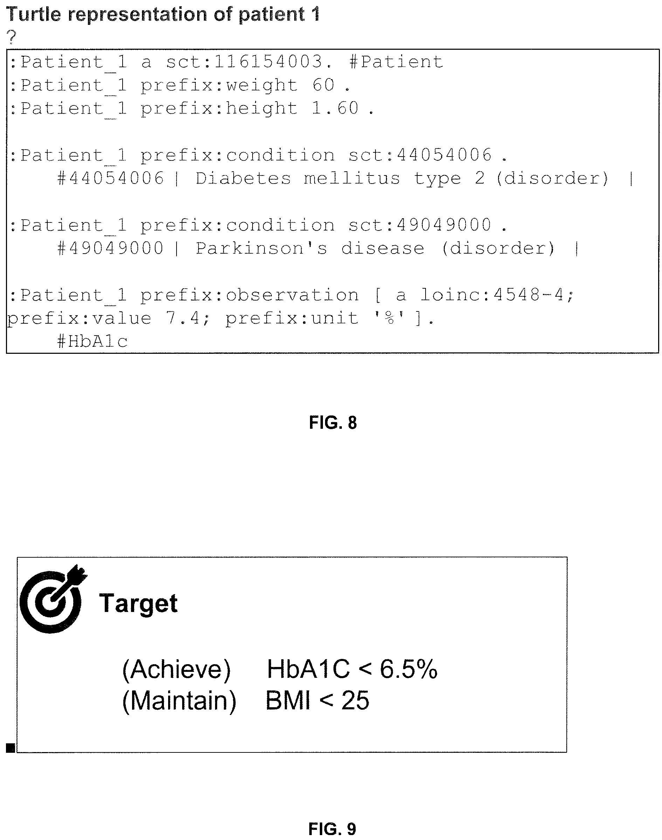

Figures 4 to 7 illustrate the method by means of an example. Figure 8 is the Turtle representation of patient 1.

The example of figures 4 to 7 demonstrate how the proposed method provides different alerts properly in diabetes treatment.

The four pictures of figures 4 to 7 show three patient profiles, all aim for treatment of diabetes.
The profiles of the three patients are rather similar, the difference is that patient 2 has a condition of Myocardial Infarction, patient 3 has a lighter weight, but the BMI still falls in normal range, and patient 4 has a heavier weight, and the BMI of patient 4 would be considered as overweight because it is greater than 25.

The target is to provide treatment to the new condition Diabetes.

The direct target is to lower the HbA1c, which is a measure for estimating the average blood glucose over the past three months. The target is to achieve HbA1c less than 6.5%.

Obesity is generally known and will have bad consequences for diabetes patient. Although all three patients are not obese in the situation illustrated in figures 4 to 7, it is an additional target to keep the BMI value below 25.

Figure 9 schematically illustrates this target.

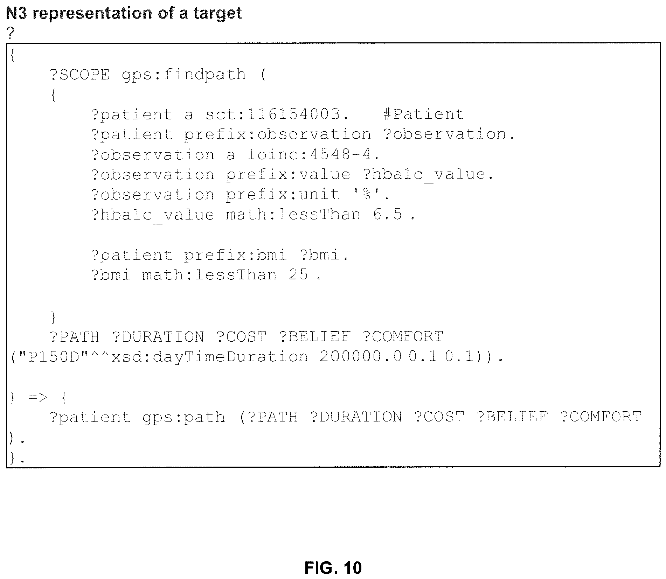

Figure 10 is a N3 representation of the target.

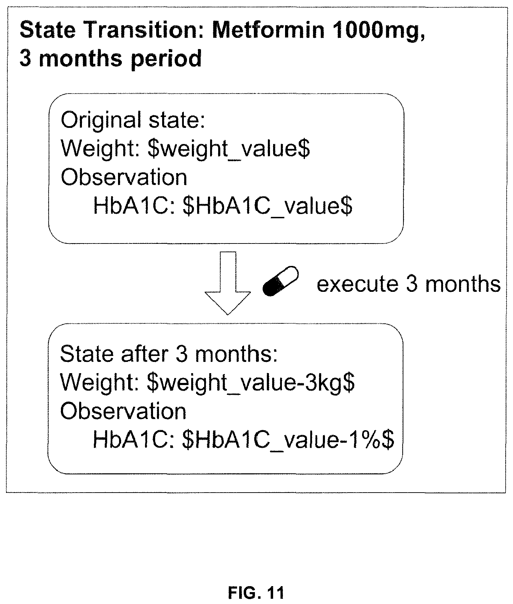

Metformin is nowadays the first choice of treatment for type II diabetes in many recommendations. The method proposed in this patent is to formally describe the consequence of actions so as to predict the future state. The consequence of the treatment with Metformin is described in Figure 11.

When Metformin is administered to the patient with the dosage of 1000 mg, the consequence after a treatment period of three months is that the HbA1c value would be reduced by 1% [reference1, page 1862] and the weight value would also decrease with 3 kg [reference 2, page 1525].

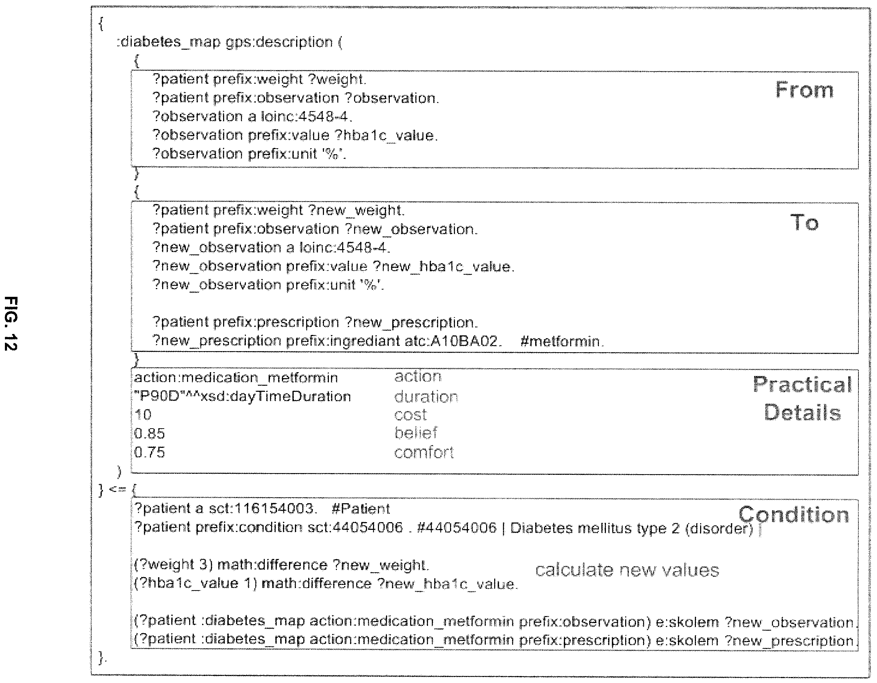

The 'From' part retracts the existing value, and the 'To' part asserts the new value. Besides stating the condition that allows to perform the state transition (that a patient is diagnosed as Type II diabetes), the condition section also calculates the new values to be asserted. Besides stating the 'From', 'To', and 'Condition' as stated in Figure 1, the example shown in the table of figure 12 also contains some practical details: 'action' indicates the action associated to the state transition; 'duration' indicates the period of the state transition; 'cost' indicates the cost of the action; 'belief' indicates the confidence of the state transition; and 'comfort' indicates the comfortness of the action. These practical details can be used in calculating and evaluating a care path.

### Reference:

Sherifali D, Nerenberg K, Pullenayegum E, et al. The effect of oral antidiabetic agents on A1C levels[J]. Diabetes care, 2010, 33(8): 1859-1864.
Maayan L, Vakhrusheva J, Correll CU. Effectiveness of medications used to attenuate antipsychotic-related weight gain and metabolic abnormalities: a systematic review and meta-analysis. Neuropsychopharmacology. 2010; 35(7):1520-1530.

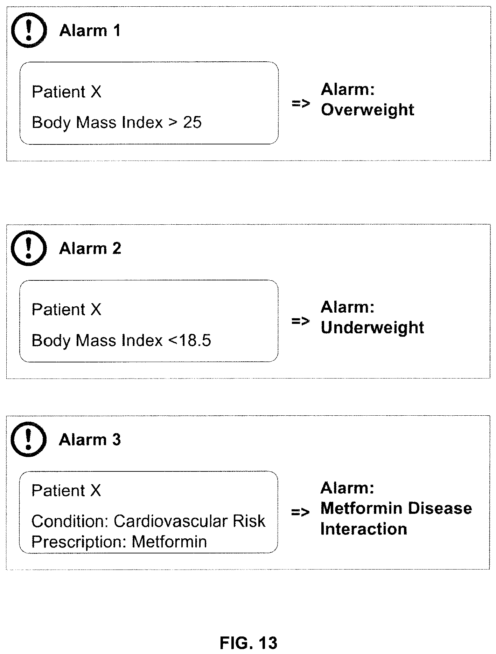

As background knowledge, a set of rules are set up to detect situations where an alert should be triggered. Figure 13 shows three type of alerts: overweight alarm, underweight alarm and Metformin Disease Interaction.

Reference: https://www.drugs.com/disease-interactions/metformin.html

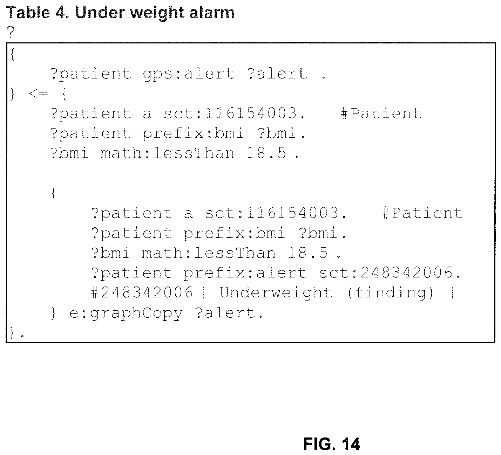

The table of figure 14 shows the N3 representation of the underweight alarm. If a patient is detected with BMI value less than 18.5, the subject graph of the e:graphCopy predicate will be copied as an alert. It also indicates the alert as sct:248342006, which is a formal representation of clinical finding 'underweight'.

But as can be observed, the patient profiles does not directly provide BMI, and none of the patient is stated with condition of Cardiovascular Risk. So none of the alarm will be triggered.

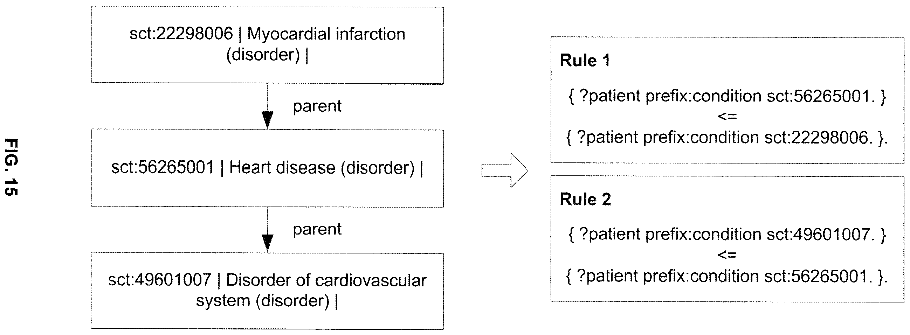

Also as background knowledge, a set of inference rules are set up so as to allow inference knowledges which are not explicitly stated. Examples are given in Figure 15.

Figure 15 shows a set of SNOMED hierarchical rules. The left side of Figure 15 stated the fact that the SNOMED concept 22298006 | Myocardial infarction (disorder) | has a parent concept 56265001 |Heart disease (disorder) |. In the meantime, the concept 22298006 | Myocardial infarction (disorder) | also has a parent
concept 49601007 | Disorder of cardiovascular system (disorder) |.

The right side of Figure 15 states two corresponding rules: if a patient is having the condition myocardial infraction (22298006), the patient is also considered as having the condition heart disease (56265001); if a patient is having the condition heart disease (56265001), the patient is then also considered as having the condition disorder of cardiovascular system (49601007).

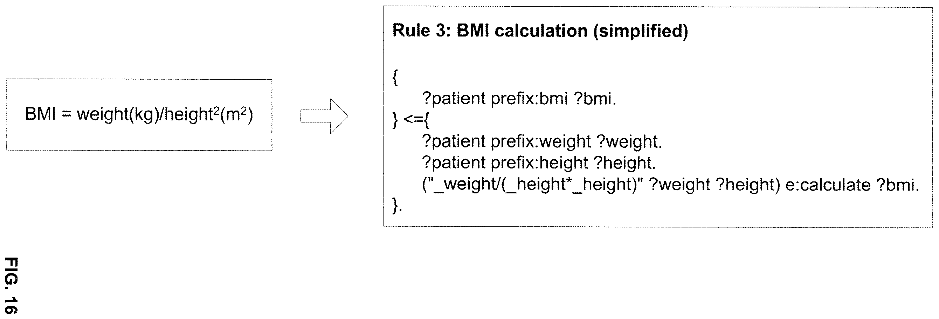

Figure 16 shows a simplified BMI calculation rules. The right side of Figure 16 shows a backward rule that generates BMI values for a patient upon request, based on the weight and height of the patient. In practice, the rule also needs to take into account of the age of the patient, and the measurement date of weight and height so as to guarantee the correctness of the calculated BMI value. Applying the inference rules to the four patient profiles shown in Figure 1, the BMI values of these four patients are calculated.

Take a patient profile stated in Figure 1 (represented in Turtle representation as displayed in the table of figure 8), apply the inference rules in Figure 15 and Figure 16 to inference BMI and SNOMED code, together with the state transition rule stated in the table of figure 12 to predict the future state, together with the alarm detection rules in the table of figure 14, executed by the EYE reasoner, the system is able to detect alerts either with the current state or predicting an alert of future state (by retracting and asserting the graphs stated in the state transition rule). EYE is an open source N3 reasoner [https://github.com/josd/eye] .

As stated before, the BMI of patient 4 is greater than 25, which would trigger an alert without any state transition.

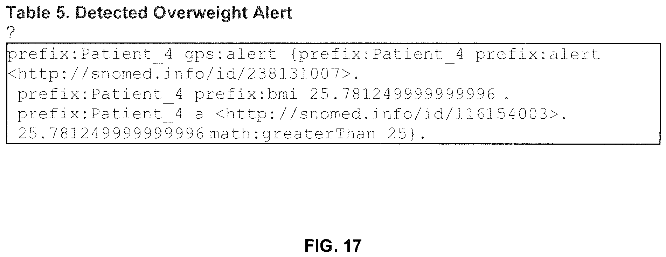

The table of figure 17 shows the detected alert. The content presented in the table of figure 17 is generated by the EYE reasoner. The result can be processed by a parser to provide a better view. The alert detected in the table of figure 17 indicates that there is an overweight alert (SNOMED concept 238131007) with the current state of patient 4 because the BMI of Patient 4 is 25.78, which is over 25.

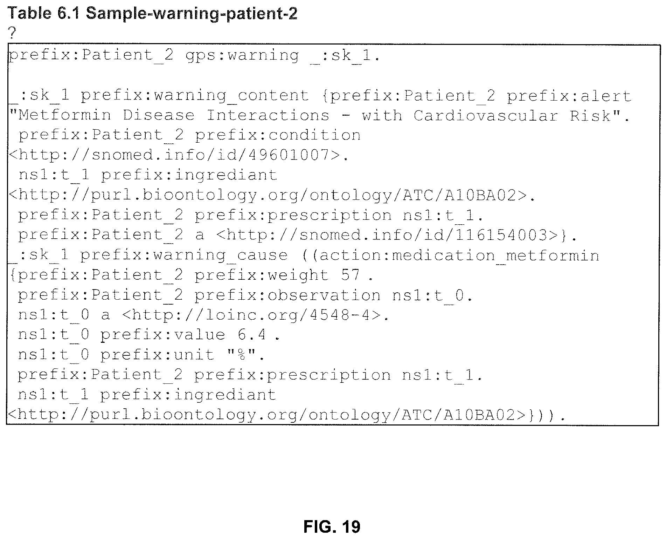

One important feature of the proposed patent is to predict a future state. Applying the state transition in Figure 3 to patient 1, 2 and 3, the future state can be predicted as described in Figure 19.

The values in grey shows the state transition introduced by taking Metformin, as stated in Figure 3. The fact that Metformin is prescribed is also asserted as a fact. It appears to be good that all these three patients have their HbA1c value under 6.5% and the BMI is also maintained under 25. However, if patient 2 takes the medication Metformin, the system will detect an alert: "Metformin Disease Interactions - with Cardiovascular Risk" because the patient has the condition of disorder of cardiovascular system (49601007), and at the same time takes medication Metformin. The table of figure 19 shows the detected alert as generated by the EYE reasoner.

Because the alert is detected based on a future state that is not in reality yet: the prescription Metformin is not administered and the alert is then considered as 'warning'. The cause of the warning can be disclosed in a warning text as shown contained in the table of figure 19. It is stated that the cause is the action:medication_metformin, which predicts the fact that the patient will have a prescription that contains ingredient Metformin (ATC code A10BA02).

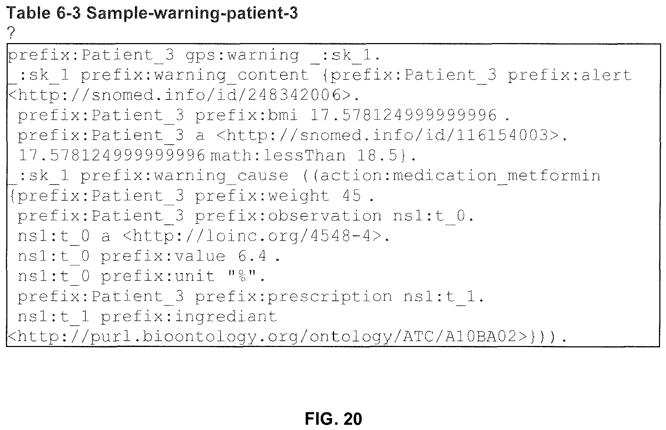

The table of figure 20 shows a warning of possible consequence of being underweight in future for patient 3. It indicates the warning is an underweight warning (<http://snomed.info/id/248342006>), because the BMI 17.578 is less than 18.5. It also explains the cause of this warning: by taking the action metformin
(action:medication_metformin), the weight of this patient become 45 (kg).

Having described in detail preferred embodiments of the current invention, it will now be apparent to those skilled in the art that numerous modifications can be made therein without departing from the scope of the invention as defined in the appending claims.

## Claims

1. A computer-implemented method of predicting and alerting a health risk in an aggregated clinical carepath prescribing a number of state transitions resulting from medical interventions prescribed in said aggregated care path comprising the steps of
- loading in a state transition engine a knowledge data base comprising said aggregated clinical carepath and a set of state transition descriptions expressed as facts and/or as rules,
- loading in said state transition engine an alert data base comprising a description of abnormal medical situations requiring an alert expressed as facts and/or as rules
- importing into said state transition engine data representing a current health state of a patient expressed as facts and/or as rules,
- performing in said state transition engine state transitions described in said aggregated carepath in sequence to simulate a state transition predicted in said carepath,
- checking by the state transition engine whether an abnormal situation requiring an alert as provided in said alert data base occurs during this simulation,
- if an alert situation is detected, generating said alert by means of said state transition engine,
- if no alert situation is detected, continuing to simulate by means of said state transition engine state next transitions of said aggregated carepath and again checking whether an abnormal situation occurs on the result of said state transition,
- repeating detection step and transition steps until all state transitions of said aggregated carepath have been performed.

2. A method according to claim 1 wherein said facts and/or as rules can be expressed in semantic web language.

3. A method according to claim 1 wherein EYE is used as rule engine to execute said state transitions.

4. A method according to claim 1 wherein Linear State Transition Logic is used separating conditions from facts.

5. A method according to claim 1 wherein an alert of an abnormal situation is one of an indicator that a vital sign is out of range, a temporal constraint, an abnormal current situation of a patient.
